# EUROPEAN PATENT APPLICATION

(11) **EP 1 619 241 A1**
(43) Date of publication of application: **25.01.2006**
(21) Application number: 05104777.7
(22) Date of filing: 02.06.2005
(51) Int. Cl.: C12N 1/21, C12N 15/74

(54) **Genetic modification system for myxobacteria**

(30) Priority: 23.07.2004 EP 04103546
(71) Applicant: Gesellschaft für Biotechnologische Forschung mbH, 38124 Braunschweig (DE)
(72) Inventor: Irschik, Herbert, 38302, Wolfenbüttel (DE); Gerth, Klaus, 38124, Braunschweig (DE); Kopp, Maren, 66121, Saarbrücken (DE); Perlova, Olena, 66119, Saarbrücken (DE); Müller, Rolf, 66440, Blieskastel (DE)
(74) Representative: Taruttis, Stefan Georg

(57) **Abstract**

The present invention provides a method for transforming myxobacteria by electroporation, thus eleminating the need for conjugation to transfer DNA. As a result, DNA to be transferred into myxobacteria does not need to contain sequences which are interacting with and form part of the conjugation system. Furthermore, transformed myxobacteria of the genus Sorangium are provided which do not contain DNA sequences which form part of the conjugation system.

## Description

The present invention relates to the genetic modification of myxobacteria, especially of the genus *Sorangium.* In greater detail, there are provided a method for genetically modifying myxobacteria, which is also useful for isolating DNA sequences from these bacteria, as well as a method for creating a library of myxobacterial DNA. Furthermore, the present invention provides the myxobacteria carrying the genetic modification introduced by the inventive method and a library made using the inventive method.

Myxobacteria, especially of the genus *Sorangium* are known producers of secondary metabolites which have a potential to have antibiotic or other biological activity, which activities make them interesting substances or lead structures for pharmaceutical use. Therefore, methods for efficient transformation are necessary for identifying gene products participating in the synthesis of such metabolites.

The investigation of and manipulation of Myxobacteria, especially of the genus *Sorangium,* is hampered by the fact that they cannot at present be transformed using standard transformation procedures. Until today, the transfer of foreign DNA into *Sorangium* is performed by making use of conjugation with *E*. *coli.* As one precondition for transforming DNA from *E*. *coli* into another bacterial cell by conjugation, subsequently also termed transconjugation, there is the presence of the structural genes encoding the conjugation apparatus of *E. coli,* namely the *tra-*genes encoding the bacterial pili, present for example on the resistance transfer factor region (RTF) of resistance plasmids. The DNA construct to be transconjugated into the recipient cell necessarily includes an oriT region, conferring onto the DNA construct the ability to be transported by conjugation. One practical difficulty in transconjugating Myxobacteria, especially *Sorangium,* is to determine the optimum conditions, e.g. the ratio of cell numbers for *Sorangium*/*E. coli.*

As Myxobacteria do not stably support DNA in the form of extrachromosomal plasmids, DNA which is introduced has to be integrated into the bacterial genome for preservation. For integration of DNA into the myxobacterial genome, two mechanisms are employed:
1. Transposition, which is the transposase mediated integration of a DNA portion that is framed by inverted terminal repeat sequences (ITRs). This process is dependent on the activity of transposase and on the presence of ITRs framing a DNA portion which is desired to be integrated into the myxobacterial genome. The integration randomly occurs and leads to the integration of the DNA portion flanked by ITRs, including the latter sequence elements. When using transconjugation in combination with the transposase mediated integration to introduce DNA into Myxobacteria, the tra genes can be physically separated from the DNA to be transconjugated and there is no requirement for presence of the oriT element between the ITRs as long as the oriT is physically linked to the DNA intended for transconjugation.
2. In the alternative, homologous recombination is used and the DNA to be introduced comprises portions homologous to the myxobacterial genome. As a result of the recombination, the DNA introduced into the myxobacterial genome is flanked by substantially identical sequence portions, one originating from the myxobacterial genome, the other from the homologous portion introduced. The differences between these flanking and substantially identical homologous sequence portions essentially correspond the differences between the myxobacterial genomic copy of that portion and that of the introduced copy. As a consequence of transconjugation, an oriT element is mandatorily contained on the DNA intended for transconjugation, which oriT is integrated into the myxobacterial genome as a consequence of homologous recombination occuring at the substantially homologous DNA portions.

### State of the Art

The transfer of plasmids into *Sorangium cellulosum,* one representative of Myxobacteria, using conjugation from *E. coli* is described by Jaoua et al. in *Plasmid* 28, 157-165 (1992). The DNA construct to be transferred into the myxobacterium includes the transposon Tn5, containing the transfer origin oriT of the 1.9 kb Sau 3A fragment RP4-mob of RP4. This oriT element results in the mobilizable characteristics of the plasmid. The inclusion of fragments from genomic *Sorangium* DNA resulted in integration of the DNA construct, presumed to occur at homologous sites.

For conjugation, in the case of self-transferrable plasmids, Jaoua et al. used *E. coli* donor strain W3101. In the case of non-self-transferrable plasmids, *E. coli* donor strain W3101 carried an additional helper plasmid (pME305) supplying the transfer functions *in trans.* Accordingly, in each case the *E. coli* donor comprised the structural genes necessary for transfer, either as a portion of the plasmid to be transconjugated, or *in trans* when encoded by the helper plasmid. The transconjugated plasmid always contained an oriT, part of the RP4-specific mobilization site, as a funcional component necessary for transconjugation.

Reported transfer efficiencies were determined by selection for antibiotic resistance to 1-5 x 10⁻⁶ to 10⁻⁷ per viable recipient cell. As an optimization step, a heat shock at 50 °C for 10 minutes led to increased transfer frequencies of 1-5 x 10⁻⁵.

Pradella et al., *Arch. Microbiol.* 178, 484-492 (2002) describe a transfer method for plasmids into *Sorangium* by conjugation with *E. coli.* For conjugation, two *E. coli* strains, each containing part of the RP4 conjugation system (Guiney, in *Bacterial Conjugation,* Clewell (eds.), Academic Press, London, 75-103 (1993)), were used contemporaneously to transconjugate plasmid DNA into *Sorangium cellulosum.* One of the *E. coli* strains (ET12567) harbours conjugation plasmid pSBB40 to be transconjugated into *Sorangium,* which plasmid contains the RP4 oriT for mobilization and the hygromycin-resistance cassette as a selection marker. The other *E. coli* strain (HB101) harbours plasmid RK600 which carries the functions necessary for mobilization, RK2-Mob and RK2-Tra, as well as the resistance to chloramphenicol. Transconjugation efficiencies of approximately 10 colonies are reported when using 100 µL *Sorangium* at a cell density of 10⁹ cells/mL in the triparental mating experiments.

From Kopp et al., *J. Biotechnology,* 107, 29-40 (2004), a method for transconjugation of plasmid sequences into the genome of *Sorangium cellulosum* is known. The plasmid transconjugated into *Sorangium* comprised the oriT from plasmid RP4 as a functional element. For analysis of the genome of *Sorangium,* a mariner-based transposon containing the hygromycin resistance cassette as a selection marker was constructed which allowed selection of transcojugated cells and subsequent extraction of DNA regions adjacent the integrated transposon sequence. However, Kopp et al. demonstrated that experimental conditions for effective transconjugation had to be adapted when transconjugating another strain of *Sorangium cellulosum,* making conjugation a complicated method for genetic manipulation of at least *Sorangium.*

WO2004/015088 discloses a transposon based vector for transforming Sorangium, the vector comprising inverted terminal repeat (ITR) sequences and a transposase gene under the control of a T7A1 promoter to carry any exogenous DNA arranged between the ITR sequences. The examples demonstrate the necessity of the presence of an oriT region of RP4 for transconjugating a DNA construct into *Sorangium.* According to the description, the oriT region is contained in the transconjugated DNA construct for the purpose of conjugating the final plasmids from *E*. *coli* into *Sorangium,* making reference to the method of Jaoua (loc. cit.). Although electroporation is generally mentioned as a possibility to transfer DNA into *Sorangium,* no details are given.

The methods according to the state of art suffer from the drawbacks of necessarily adapting the experimental conditions for conjugation, even between strains of one genus, and of yielding rather low rates of genetically modified myxobacteria, e.g. low efficiencies of DNA transfer by conjugation. Furthermore, the transfer of DNA constructs by conjugation necessitates the presence of an oriT as part of the construct and, in addition, the presence of the transfer functions, namely the products of the *tra*-genes.

The use of electroporation for transferring DNA into *Sorangium* has hence been impossible.

The standard electroporation protocols do not yield transformants. Practical difficulties arising when attempting transformation of *Sorangium* are for example due to the slime secreted by these bacteria and the formation of aggregates.

Kimsey et al., *J. of Bacteriology* 173, No 21, 6790-6797 (1991) describe the transformation of *Myxococcus xanthus* by electroporation with a circular plasmid. Conditions for electroporation were growing *M xanthus* to exponential phase in the rich growth medium CTT broth, collecting cells by centrifugation (10,000 x g for 5 min) and 2 to 4 washes in electroporation buffer (270 mM sucrose, 5 mM KPO₄, pH 7.6, 15 % glycerol), then mixing chilled cells at 200 µL with 1 to 5 µg supercoiled plasmid DNA and immediately applying an electric field pulse of 5,000 V/cm at a capacitor setting of 25 µF at time constants of 2 to 10 ms in a BioRad GenePulser apparatus. Following electroporation, the cell suspension was added to 3 mL molten CTT soft agar and plated on CTT agar containing 40 µg/mL kanamycin sulfate. After 5-7 days incubation at 32 °C, kanamycin resistant transformants could be isolated.

### Objects of the invention

In view of the known state of art, the present invention seeks to overcome the drawbacks of the state of art, especially of transconjugating DNA into myxobacteria, for example of the genus *Sorangium.* As a specific object, the present invention seeks to provide a method for electroporating myxobacteria, especially *Sorangium,* which is unknown to-date.

### General description of the invention

The present invention achieves the above objects by providing a novel method for transforming myxobacteria using electroporation. This method is especially suitable for the order *Myxococcales,* preferably the genus *Polyangium,* also termed *Sorangium,* and more preferably for the cellulolytic strains thereof, also termed *Sorangium cellulosum,* for example the strains available under accession numbers ATCC25531, ATCC29479 (DSMZ 2044).

Furthermore, the present invention provides genetically modified *Sorangium* obtainable as the result of the novel transformation method. Examples of genetically modified *Sorangium* include transformants in which specific pathways for synthesis, for example synthesis of secondary metabolites like polyketides, e.g. etnangien or chivosazole, are interrupted. Such synthesis negative transformants are useful for identifying the gene cluster and structural genes participating in the synthesis of said secondary metabolites.

In a further aspect, the present invention provides a novel system for obtaining a genomic library of *Sorangium,* preferably a partial library enriched for pre-selected regions, which can be obtained by using the inventive transformation method. In general, one step of generating a genomic library according to the invention comprises the transfer of a DNA construct into *Sorangium.* By pre-selecting sequences of the DNA construct to show homology to sequences of desired functions of *Sorangium,* an enrichment or pre-selection of the resultant partial library for desired sequence regions is achieved. Within the context of this disclosure, the terms "genomic library" and "partial library" not only refer to specialized genetic systems capable of containing large DNA fragments in large numbers, but also to any number of host organisms, e.g. bacteria like *E*. *coli,* harbouring the DNA construct transformed into myxobacteria and re-isolated from myxobacterial transformants, which preferably are fused to genomic DNA fragments recovered from the myxobacterium.

Genomic libraries according to the invention can for example be generated using the transposon mediated integration of DNA transformed into myxobacteria, especially of the genus Sorangium. For this purpose, the transposase activity is used, resulting in a random integration of DNA of the myxobacterial genome, therefore essentially leading to a statistical distribution of integrated DNA flanked by ITRs across the entire genome. For example, the *mariner-based* transposon preferentially integrates at T/A sites.

For generating a genomic library exemplary DNA constructs used for transformation of myxobacteria according to the invention comprise a selection marker that is expressed and functional in myxobacteria for selection of transformants, for example the hygromycin resistance cassette when transforming *Sorangium.* Furthermore, such DNA constructs can comprise the essential sequences of a transposon, e.g. ITRs coding for transposase which is functional in the relevant myxobacterium. An example for the transposon sequence is the *mariner-based* transposon comprising the hygromycin resistance gene under control of the aph II promoter and a transcriptional terminator, arranged between inverted terminal repeat (ITR) sequences. The ITR sequences can for example be derived from Tn5.

In contrast to genomic libraries, partial libraries enriched or pre-selected for certain regions can be obtained by using homologous recombination between the myxobacterial genome and the transformed DNA. The selection is caused by the preferential integration at sites homologous to the transformed DNA sequence.

A method for creating an enriched or pre-selected genomic library as well as the resultant library itself, according to the invention utilizes the preference of DNA constructs transferred into myxobacteria to integrate at regions of homology. Accordingly, the DNA construct transferred into myxobacteria comprises a sequence of homology to the myxobacterial genome encoding the desired functions.

For generating a genomic or partial library, subsequent to the transformation of myxobacteria, especially *Sorangium,* total DNA is isolated. This DNA is fragmented, for example by restriction. As an optional step, these fragments can be re-ligated. Regions adjacent the integration site of the transformed DNA construct, for example regions homologous to sequences encoding the desired functions, can be amplified by transformation into a host organism supporting maintenance of the transformed DNA construct and applying selection pressure for the selection marker contained on the DNA construct. As a convenient host, *E. coli* can be used. Identification of sequences originating from the genome of myxobacteria can now be performed according to standard practices on the plasmids contained in the host harbouring the fusion of the DNA construct which was originally transformed into myxobacterium with the myxobacterial DNA adjacent its integration site.

The inventive method for transforming myxobacteria is independent of the conjugation system. Accordingly, the present invention allows the genetic modification of myxobacteria without necessity for the functions of the tra-genes and without necessity for transconjugation specific genetic elements present on the DNA construct to be transformed into myxobacteria. One example for such transconjugation specific genetic elements is the oriT element. Accordingly, the resultant transformants do not necessarily contain an oriT element which stems from the transformed DNA construct.

As a result, myxobacteria, especially *Sorangium,* transformed by the method according to the present invention need not contain an oriT. Furthermore, DNA recovered from fragmented genomic DNA of myxobacteria which have been transformed the method according to the present invention, need not contain an oriT.

The electroporation method according to the present invention uses cells which are obtainable from liquid culture. These cells are harvested by centrifugation and resuspended in a specific washing buffer at 0 to 5 °C. After washing, cells are resuspended in a specific electroporation buffer at 0 to 5 °C, mixed with added DNA, and subjected to an electric field pulse. The washing buffer consists of a low ionic strength aqueous solution at neutral to basic pH, preferably at pH 7 to 8, containing at maximum 5 % by weight, preferably at maximum 1 % by weight, more preferably at maximum 0.5 % by weight, of a non-ionic polymer, preferably a carbohydrate, most preferably a sugar.

The present inventors have discovered that transformation by electroporation is only possible when using a suspension of *Sorangium* in medium which is essentially free from divalent cations. Furthermore, it has been discovered that following the washes of cultivated cells, in washing buffer, efficient electroporation of cells suspended in the washing buffer cannot be obtained. It has been found that electroporation can be carried out efficiently after changing the washing buffer to a specific electroporation buffer, which is essentially free of ionic compounds. Preferably, the electroporation buffer is an aqueous solution of a carbohydrate, preferably of up to 5% by weight, up to 2.5% by weight, more preferably up to 1% by weight, and most preferably up to 0.5% by weight. Carbohydrates can be selected for sucrose, glucose, mannose, maltose, fructose, galactose, and dimers, trimers and polymers thereof as well as mixtures of these compounds with the preferred carbohydrate being glucose.

Electroporation is carried out with the cells suspended in special electroporation buffer, containing at maximum of 5 % by weight, preferably at maximum 1 % by weight, more preferably at maximum 0.5 % by weight, of a non-ionic polymer, preferably a carbohydrate, most preferably a sugar, preferably selected from the group consisting of sucrose, glucose, mannose, fructose, galactose and dimers, trimers and polymers thereof, as well as mixtures of the aforementioned compounds. It is preferred that the washing buffer and the electroporation buffer are essentially free of divalent cations, e.g. calcium ions. Essentially free of divalent cations means a concentration of below 10 mM, preferably below 1 mM, more preferably below 0.1 mM or below 10 µM and most preferably below 1 µM.

Preferably, subsequent to subjecting the cells to the electric field pulse, cultivation medium without selection marker is used for cultivating the cells for 10 to 30 hours at 30 °C, then optionally changing the medium to antibiotic containing selective liquid medium and incubating for an additional 8 to 20 hours, optionally with agitation corresponding to shaking of culture flasks at 100 to 300 rpm, preferably at 150 to 250 rpm. Following the incubation in non-selective liquid medium with the optional additional incubation in liquid selective medium, transformed cells are plated onto solid selective medium. Prior to plating, cells can be pelleted by centrifugation and resuspended in 1 to 2 mL liquid medium, then plated in dilutions of 1:5 to 1:1000 on selective medium.

The present invention provides a novel method for genetically modifying myxobacteria by transformation using the fast and efficient electroporation method. Accordingly, no preparation of donor cells, for example *E*. *coli,* is necessary and the supply of the conjugation machinery, for example the products of the tra-genes, can be omitted, leading to a greatly simplified method for genetically modifying myxobacteria. As a consequence of electroporation, DNA to be transformed into myxobacteria can be free from sequences or genetic elements that interact with or form part of the conjugation system of *E. coli,* which was employed hitherto.

According to the invention, the DNA construct transformed into the myxobacterium for creating a library is devoid of sequences originating from bacterial conjugation, for example it carries no oriT from the *E*. *coli* conjugation system.

As a further advantage, the present invention provides a higher number of genetically modified myxobacteria, resulting from the higher efficiency of transformation by electroporation in comparison to the efficiency of conjugation. This relates to the number of modified myxobacteria per number of myxobacteria used for transformation or conjugation, respectively, as well as to the number of modified myxobacteria per amount of DNA construct used. Experimental transformation efficiencies obtained a range between 6 - 8 x 10⁻⁷ transformants per cells used for electroporation at DNA amounts of 1 to 10 µg.

The method according to the invention for genetically modifying myxobacterium comprises the steps of
a) cultivating the myxobacterium in liquid culture,
b) harvesting the cells by centrifugation,
c) washing the harvested cells two to four times in low ionic strength washing buffer,
d) collecting the washed cells by centrifugation,
e) re-suspending the cells in electroporation buffer essentially free of ionic compounds,
f) electroporating the cells with a DNA construct devoid of genetic elements of a bacterial conjugation system.

In addition to the aforementioned steps, the method for genetically modifying myxobacterium can be characterized in that the washing buffer washing buffer is water with a maximum content of up to 10 mM, preferably up to 5 mM, more preferably up to 2 mM of a non-ionic buffering compound at a pH of 7 to 8 and a carbohydrate of up to 5% by weight, preferably up to 2.5 % by weight, more preferably up to 1% by weight,
optionally further characterized in that the electroporation buffer is an aqueous solution of up to 2.5 % by weight, preferably of up to 1% by weight, more preferably of up to 0.5% by weight of a carbohydrate,
optionally further characterized in that the carbohydrate of the washing buffer and/or the electroporation buffer is selected from the group consisting of sucrose, glucose, mannose, fructose, galactose and dimers, trimers and polymers thereof as well as mixtures of the aforementioned compounds.

In addition to the aforementioned steps, the method can be characterized in that electroporation is carried out at 850 - 2000 V, a resistance setting of 600 - 100 Ω, a capacitor setting of 10 - 25 µF using a gap width between electrodes of 0.1 - 0.2 cm,
optionally further characterized in that cells are suspended at a density of 1 x 10⁹ to 5 x 10⁹ cells/mL in electroporation buffer,
optionally further characterized in that the DNA construct comprises a transposon or a region of homology,
optionally further characterized in that following electroporation cells are cultivated in non-selective liquid medium for 10 - 30 hours.

Further, the present invention provides a method for creating a genomic library of myxobacterium, which in addition to the above mentioned steps comprises the steps of
g) cultivating the transformed myxobacterium,
h) isolating total DNA from the transformed myxobacterium,
i) fragmenting the isolated DNA,
j) transforming the fragmented DNA into a host organism and
k) selecting for host organisms transformed with DNA comprising the DNA construct.

Preferably, the myxobacterium for creating a genomic library belongs to the genus *Sorangium.*

The method for creating a genomic library can also be characterized in that the DNA construct comprises a transposon, which may be *mariner-based.*

Further, the method for creating a genomic library may be characterized in that the DNA construct comprises a region of homology.

As a result of the method for creating a genomic library, the genomic library obtainable is part of the invention.

The present invention also provides myxobacteria of the genus *Sorangium* which are genetically modified by the inventive method for genetical modification, i.e. by electroporation.

### Detailed description of the invention

The invention will now be described in greater detail by way of examples using the myxobacterium *Sorangium cellulosum,* strains So ce 56 and So ce 12. Due to the heterogeneity of the genus *Sorangium,* electroporation conditions might need optimization to account for differences between individual strains. Such adaptations for optimization for individual strains are considered to be within the capabilities of the skilled person. Percentages are in percent by weight unless otherwise indicated.

### Example 1: Electroporation of Sorangium cellulosum

*Sorangium cellulosum* strain So ce 56 was cultivated in shake flasks at 30 °C at 160 rpm in M-medium containing 1.0 % soy tryptone, 1.0 % maltose, 0.1 % CaCl₂, 0.1 % MgSO₄·7H₂O, 50 mM HEPES and 8 mg/L Na-Fe-EDTA, adjusted to pH 7.2. Alternatively, SM-medium was used, containing 5 g/L asparagine, 0.5 g/L MgSO₄·7H₂O, 100 mM HEPES, 10 mg/L Fe-EDTA, 0.5 g/L CaCl₂, 0.06 g/L K₂HPO₄, 10 g/L maltose.

When a cell density of 10⁸ to 10⁹ cells/mL was reached, cells were harvested by centrifugation using a GS3 rotor at 6,000 rpm at 0 to 5 °C.

All subsequent steps were carried out at 0 to 10 °C, preferably below 5 °C or on ice. The cell pellet was washed two to four times by resuspending the cells in washing buffer and subsequent collected by centrifugation using an SS34 rotor at 10,000 rpm. The washing buffer consisted of 1 mM HEPES (N-2-hydroxy ethyl piperazine-N'-2-ethane sulfonic acid) and 0,5 % by weight glucose at a pH of 7.4 in distilled pure water.

Following the washes, the cell pellet was resuspended in electroporation buffer, consisting of 0.5 % glucose in distilled pure water. The cells were resuspended to a cell density of 1 x 10⁹ - 5 x 10⁹ cells/mL and aliquotted to 100 - 300 µL for storage on ice until used for electroporation. Immediately prior to electroporation, DNA was added and mixed with the cells in an Eppendorf cup, then the suspension was transferred into an electroporation cuvette.

The DNA construct for transformation contained transposon sequences comprising an expression cassette for transposase arranged between terminal repeat sequences and a DNA region with homology to genomic regions of *Sorangium.*

Electroporation was performed after adding DNA to be transferred into Sorangium at a concentration of 5 - 80 µg/mL cell suspension. The amount of DNA was 1 to 8 µg in the form of a plasmid. For electroporation, 100 to 300 µL cell suspension in electroporation buffer including the DNA are used at a voltage of 850 - 2000 V, a resistance setting of 600 -100 Ω, and a capacitor setting of 10 - 25 µF. The cuvette used had a gap width of 1 - 2 mm between electrodes. Time constants generally were 2 to 10 ms, preferably 4 to 8 ms.

Following the electroporation, the cell suspension was transferred into 10 mL liquid culture medium M or SM without selection marker and incubated in shake flasks for 10 - 30 hours at 30 °C and shaking at 170 rpm. Subsequent to this expression phase, the medium was changed to selection-marker containing medium, namely antibiotic containing M- or SM-medium, and cultivated for 8 to 20 hours at 30 °C under 170 rpm movement in shake flasks. Medium change was done either by adding the relevant antibiotic or by collecting the cells by centrifugation according to the harvesting and resuspending the pelleted cells in antibiotic containing medium.

For plating, cells were again collected by centrifugation as before and resuspended in 1 - 2 mL M- or SM-medium and spread on agar plates of P-agar containing 0.2 % peptone (Marcor), 0.8 % starch (Merck, Darmstadt, Germany), 0.4 % probion (Gerth, et al., (1984)), 0.2 % yeast extract (Difco), 0.1 % CaCl₂·2H₂O, 0.1 % MgSO₄·7H₂O, 2.4 % HEPES (Roth, Karlsruhe, Germany), 8 mg/L NaFe-EDTA, and 1.5 % agar. The pH was adjusted to 7.5. After autoclaving, the medium was supplemented with 0.2 % glucose ·H₂O. Incubation of agar-plates was done for 10 - 20 days at 30 °C.

### Example 2: Electroporation of Sorangium cellulosum

Strain So ce 12 was treated as described in Example 1, except for cultivation HS-medium was used. HS-medium contains 0.1 % MgSO₄·7H₂O, 0.1 % KNO₃, 0.15 % peptone form casein (Merck, Darmstadt, Germany), 0.2 % TRIZMA base (Sigma Aldrich, Seelze, Germany) and 8 mg/L NaFe-EDTA. The pH was adjusted to 7.2 and supplemented with 0.00625 % K₂HPO₄, 0.4 % glucose ·H₂O, and 0.0075 % CaCl₂ ·2H₂O after autoclaving. For plating, solid medium PM₁₂ was used, containing 0.04 % peptone form casein (Merck, Darmstadt, Germany), 0.15 % MgSO₄·7H₂O, 0.2 % TRIZMA base (Sigma Aldrich, Seelze, Germany), and 1.5 % Agar (Difco, Hamburg, Germany), adjusted to pH 7.3. After autoclaving, 0.1 % CaCl₂ ·2H₂O, 8 mg/L NaFe-EDTA, 0.2 % KNO₃, 0.00625 % K₂HPO₄, 0.35 % glucose·H₂O, and 0.6 mM dithionite were added.

### Example 3: Generation of a genomic library of Sorangium cellulosum

The generation of a genomic library of *Sorangium cellulosum* was achieved by transforming cells according to example 1 or 2 with a DNA construct comprising an expression cassette for transposase and a bacterial resistance gene.

Following the plating of transformed *Sorangium cellulosum* on hygromycin containing agar plates, colonies were isolated and grown in liquid medium. Total DNA was extracted from the separate *Sorangium* cultures using the Puregene® genomic DNA purification kit (Gentra, Minneapolis, USA) according to the manufacturer's instructions. Total DNA preparations were fragmented by digestion with a restriction endonuclease not cutting the DNA construct transformed into *Sorangium,* subjected to religation and transformed into competent *E*. *coli.*

The resultant transformants were selected for by using the antibiotic hygromycin and represent a genomic library.

## Claims

1. Genetically modified *Sorangium,* obtainable by transformation comprising the steps of
a) cultivating *Sorangium* in liquid culture,
b) harvesting the cells by centrifugation,
c) washing the harvested cells two to four times in low ionic strength washing buffer,
d) collecting the washed cells by centrifugation,
e) re-suspending the cells in electroporation buffer essentially free of ionic compounds,
f) electroporating the cells with a DNA construct devoid of genetic elements of a bacterial conjugation system.

2. Genetically modified *Sorangium* according to claim 1, **characterized in that** the washing buffer is water with a maximum content of up to 10 mM, preferably up to 5 mM, more preferably up to 2 mM of a non-ionic buffering compound at a pH of 7 to 8 and a carbohydrate of up to 5% by weight, preferably up to 2.5 % by weight, more preferably up to 1% by weight.

3. Genetically modified *Sorangium* according to one of the preceding claims, **characterized in that** the electroporation buffer is water essentially free from ionic compounds containing up to 5% by weight of a carbohydrate.

4. Genetically modified *Sorangium* according to one of the preceding claims, **characterized in that** the electroporation buffer is an aqueous solution of up to 2.5 % by weight, preferably of up to 1% by weight, more preferably of up to 0.5% by weight of a carbohydrate.

5. Genetically modified *Sorangium* according to one of the preceding claims, **characterized in that** the carbohydrate of the washing buffer and/or the electroporation buffer is selected from the group consisting of sucrose, glucose, mannose, maltose, fructose, galactose and dimers, trimers and polymers thereof as well as mixtures of the aforementioned compounds.

6. Genetically modified *Sorangium* according to one of the preceding claims, **characterized in that** electroporation is carried out at 850 - 2000 V, a resistance setting of 600 -100 Ω, a capacitor setting of 10 - 25 µF using a gap width between electrodes of 0.1 - 0.2 cm.

7. Genetically modified *Sorangium* according to one of the preceding claims, **characterized in that** the DNA construct comprises a transposon.

8. Genetically modified *Sorangium* according to one of the preceding claims, **characterized in that** following electroporation, cells are cultivated in non- selective liquid culture medium for 10 - 30 hours.

9. Method for genetically modifying myxobacterium comprising the steps of
a) cultivating the myxobacterium in liquid culture,
b) harvesting the cells by centrifugation,
c) washing the harvested cells two to four times in low ionic strength washing buffer,
d) collecting the washed cells by centrifugation,
e) re-suspending the cells in electroporation buffer essentially free of ionic compounds,
f) electroporating the cells with a DNA construct devoid of genetic elements of a bacterial conjugation system.

10. Method for genetically modifying myxobacterium according to claim 10, **characterized in that** the washing buffer washing buffer is water with a maximum content of up to 10 mM, preferably up to 5 mM, more preferably up to 2 mM of a non-ionic buffering compound at a pH of 7 to 8 and a carbohydrate of up to 5% by weight, preferably up to 2.5 % by weight, more preferably up to 1% by weight.
